# EUROPÄISCHE PATENTSCHRIFT

(11) **EP 0 714 415 B1**
(45) Veröffentlichungstag und Bekanntmachung des Hinweises auf die Patenterteilung: **17.06.1998**
(21) Anmeldenummer: 94926167.1
(22) Anmeldetag: 09.08.1994
(51) Int. Cl.: C08F 2/16, C09D 157/00, A61K 7/06

(54) **WÄSSRIGE COPOLYMERISATDISPERSIONEN**
AQUEOUS COPOLYMER DISPERSIONS
DISPERSIONS AQUEUSES DE COPOLYMERES

(30) Priorität: 17.08.1993 DE 4327514
(43) Veröffentlichungstag der Anmeldung: 05.06.1996
(73) Patentinhaber: BASF AKTIENGESELLSCHAFT, 67056 Ludwigshafen (DE)
(72) Erfinder: SCHEHLMANN, Volker, D-68163 Mannheim (DE); SCHADE, Christian, D-67061 Ludwigshafen (DE); SANNER, Axel, D-67227 Frankenthal (DE); SPERLING-VIETMEIER, Karin, D-67433 Neustadt (DE)
(86) Internationale Anmeldenummer: EP9402636
(87) Internationale Veröffentlichungsnummer: WO9505402

(56) Entgegenhaltungen:
- EP-A- 0 214 626
- EP-A- 0 391 343
- US-A- 4 786 696

## Beschreibung

Die vorliegende Erfindung betrifft die Verwendung wäßriger Copolymerisatdispersionen als Filmbildner in haar Kosmetischen zubereitungen. Die wäßrigen Copolymerisat dispersionen sind erhältich durch radikalinitiierte oder durch Anwendung ionisierender Strahlung initiierte Copolymerisation von
A) 40 bis 99 Gew.-% eines oder mehrerer wasserunlöslicher, monoethylenisch ungesättigter Monomeren,
B) 1 bis 60 Gew.-% eines oder mehrerer wasserlöslicher, monoethylenisch ungesättigter Monomeren und
C) 0 bis 30 Gew.-%, insbesondere 0 bis 20 Gew.-%, eines oder mehrerer mehrfach ethylenisch ungesättigter Monomeren
in wäßrigem Medium in Gegenwart von 2 bis 20 Gew.-%, bezogen auf die Gesamtmenge der Monomeren, oberflächenaktiver Verbindungen als Emulgatoren und weisen eine mittlere Teilchengröße von 10 bis 30 nm, bestimmt durch Lichtstreuung in wäßrigem Medium, sowie einen Feststoffgehalt von 20-50 Gew.-% auf.

Durch übliche radikalische Polymerisation in dispersen Öl-in-Wasser-Phasen werden normalerweise polymere Latices mit Teilchendurchmessern von über 100 nm erhalten. Durch Polymerisation in Mikroemulsion gelangt man zu Mikrodispersionen, die in Wasser gemessene Teilchendurchmesser von unter 80 nm besitzen. Analog zu normalen Dispersionen werden diese Polymerisate auch als Mikrolatices bezeichnet.

Ökologische Aspekte machen es erstrebenswert, den Anteil flüchtiger organischer Verbindungen, insbesondere organischer Lösungsmittel, in haarkosmetischen Zubereitungen, einem der interessantesten Anwendungsgebiete für die genannten Polymerisatdispersionen, zu reduzieren. Für die Praxis bedeutet dies, daß verbesserte Haarsprays heute neben den klassischen Hauptinhaltsstoffen Alkohol, d.h. Ethanol und Isopropanol, Treibmittel und Haarfestigungsmittel (Filmbildner) zusätzlich Wasser und andere Stoffe enthalten, die nicht unter die Definition der flüchtigen organischen Verbindungen fallen. Weitere Möglichkeiten zur Reduzierung von flüchtigen organischen Verbindungen in Haarsprayformulierungen sind die Verwendung von Pumpsprays oder der Einsatz von Druckgasaerosolen. Für den Fall, daß die Formulierung Wasser enthält, wäre es unter Umweltschutz- und Kostengesichtspunkten erstrebenswert, das als Filmbildner verwendete Polymerharz in einem wäßrigen System herzustellen.

Einer technischen Umsetzung von Mikrolatices, beispielsweise auf dem Gebiet der Haarkosmetik, standen bis jetzt im wesentlichen die Probleme des zu geringen Feststoffgehaltes in den Anwendungsformulierungen solcher Mikrolatices und der sehr hohen Tensidmenge - etwa 20 bis 50 Gew.-% oder sogar noch mehr, bezogen auf die eingesetzten Monomeren - die zu ihrer Herstellung notwendig ist, entgegen. Ein weiterer Nachteil der Anwendung von Polymerisatdispersionen als Filmbildner in haarkosmetischen Zubereitungen ist die Erzeugung eines nicht gewünschten grauweißen Aussehens des Haares während der Trocknungsphase.

In der EP-B 214 626 werden Haarkosmetikzusammensetzungen mit einem teilchenförmigen Polymer beschrieben, das einen mittleren gewichtsbezogenen Teilchendurchmesser von 10 bis 100 nm und eine solche Teilchengrößenverteilung aufweist, daß die Teilchen mit einem Durchmesser von 5 bis 200 nm mehr als 95 % des Gewichtes der Teilchen ausmachen. Als Beispiel N (s. Tabelle 1 auf S. 10) wird ein Copolymer aus 94 Gew.-% Styrol und 6 Gew.-% Natriumstyrolsulfonat mit einer mittleren Teilchengröße von 42 nm beschrieben, welches durch radikalisch initiierte Emulsionspolymerisation in Wasser in Gegenwart von 20 Gew.-%, bezogen auf die eingesetzten Monomeren, des Adduktes von 30 mol Ethylenoxid an Nonylphenol als Emulgator hergestellt wurde; der herstellungsbedingte Feststoffgehalt dieser Copolymerdispersion beträgt ca. 19 Gew.-%.

Aufgabe der vorliegenden Erfindung war es, Copolymerisatdispersionen bereitzustellen, die die Mängel des Standes der Technik nicht mehr aufweisen.

Demgemäß wurden die eingangs definierten feinteiligen wäßrigen Copolymerisatdispersionen gefunden.

Erfindungsgemäß werden mittlere Teilchengrößen von 10 bis 30 nm eingesetzt vor allem von 15 bis 25 nm, bestimmt durch Lichtstreuung in wäßrigem Medium. Durch andere Bestimmungsmethoden ermittelte Teilchengrößenwerte können sich geringfügig von den hier angegebenen Werten unterscheiden, in der Regel betragen die Unterschiede zwischen den einzelnen Meßmethoden jedoch nicht mehr als 10 %.

Der herstellungsbedingte Feststoffgehalt liegt bei 20 bis 50 Gew.-%, insbesondere bei 21 bis 40 Gew.-%.

Die wäßrigen Copolymerisatdispersionen werden üblicherweise durch photochemische oder thermische Umsetzung der Monomeren A bis C in wäßrigem Medium durch Anwendung ionisierender Strahlung oder Radikale bildender Verbindungen hergestellt. Die bevorzugte Arbeitstechnik hierbei ist diejenige der thermisch radikalinitiierten Emulsionspolymerisation.

Eine bevorzugte Ausführungsform sind wäßrige Polymerisatdispersionen, welche durch Copolymerisation von
A) 60 bis 92 Gew.-%, insbesondere 70 bis 87 Gew.-% der Monomeren A und
B) 8 bis 40 Gew.-%, insbesondere 13 bis 30 Gew.-% der Monomeren B
oder durch Copolymerisation von
A) 60 bis 93,5 Gew.-%, insbesondere 70 bis 90 Gew.-% der Monomeren A,
B) 6 bis 32 Gew.-%, insbesondere 6 bis 25 Gew.-% der Monomeren B und
C) 0,5 bis 15 Gew.-%, insbesondere 0,5 bis 8 Gew.-%, bevorzugt 1 bis 5 Gew.-% der Monomeren C
erhältlich sind.

Als Komponente A kommen normalerweise solche Monomere in Betracht, die sich zu weniger als 5 Gew.-% in Wasser bei 25°C lösen. Beispiele für Monomere A sind Styrol und Styrolderivate wie α-Methylstyrol, p-Methylstyrol, p-Chlormethylstyrol, p-Butylstyrol, p-Ethylstyrol oder p-Chlorstyrol, halogenierte Verbindungen wie Vinylchlorid, Vinylidenchlorid oder Vinylidenfluorid, Vinylester von geradkettig oder verzweigten Alkylcarbonsäuren mit 2 bis 30, insbesondere 2 bis 10 Kohlenstoffatomen im Molekül wie Vinylacetat, Vinylpropionat, Vinyllaurat, Neooctansäurevinylester, Neononansäurevinylester, Neodecansäurevinylester, Butadien oder Isopren, Vinylalkylether mit 1 bis 30 Kohlenstoffatomen in der Alkylgruppe wie Vinyloctadecylether, Acrylnitril, 1-Alkene mit 3 bis 30 Kohlenstoffatomen im Molekül, N-Alkyl(meth)acrylamide mit 4 bis 30 Kohlenstoffatomen in der Alkylgruppe wie N-tert.-Butylacrylamid, N-tert.-Octylacrylamid oder N-Undecylmethacrylamid, Alkylacryl- und Alkylmethacrylester mit 1 bis 30 Kohlenstoffatomen im Molekül wie Methylacrylat, Ethylacrylat, n-Butylacrylat, iso-Butylacrylat, tert.-Butylacrylat, 2-Ethylhexylacrylat, Laurylacrylat, iso-Bornylacrylat, Stearylacrylat, Behenylacrylat und die entsprechenden Methacrylester sowie (Meth)acrylesterderivate wie Phenoxyethylacrylat, Tetrahydrofurfuryl (meth) acrylat oder Perfluoroctylacrylat. Besonders bevorzugt sind Styrol, Vinylester von Alkancarbonsäuren mit 2 bis 30 C-Atomen im Molekül, Alkyl (meth) acrylate mit jeweils 1 bis 10 Kohlenstoffatomen in der Alkylkette oder Mischungen hieraus.

Als Komponente B kommen normalerweise solche monoethylenisch ungesättigten Monomere in Betracht, die sich zu mindestens 5 Gew.-% in Wasser bei 25°C lösen. Beispielhafte Monomere B enthalten eine Gruppe aus der Klasse der Ether-, Alkohol-, Phenol-, Amino-, Imino-, Amido-, Carbonsäure-, Carbonsäureanhydrid-, Phosphonsäure-, Sulfonsäure- oder Sulfatestergruppen. Die Baugruppen können auch in konjugierter Form und/oder in Ringstrukturen integriert vorliegen. Amino- und Iminogruppen können auch durch Umsetzung mit einem alkylierenden Agens in die entsprechenden quaternären Gruppen übergeführt sein, saure Gruppen in Form ihrer Salze mit Alkali- und Erdalkalimetallen, Ammoniak oder organischen Aminen vorliegen. Beispiele für die Monomere B sind ungesättigte Carbonsäuren wie Acrylsäure, Methacrylsäure, Itaconsäure, Crotonsäure, Fumarsäure, Maleinsäure, Maleinsäure- oder Fumarsäurehalbester, z.B. Maleinsäure- oder Fumarsäuremonomethylester, -monoethylester oder -monobutylester, Phosphon- und Sulfonsäurederivate wie Vinylphosphonsäure, Natrium-vinylsulfonat, Natriumstyrolsulfonat, Acrylamidopropansulfonsäure, Kalium-Sulfatopropylmethacrylat, Ammonium-Sulphatoethylmethacrylat, Acrylamidderivate wie Acrylamid, Methacrylamid, N,N-Dimethylacrylamid-3-acrylamido-3-methyl-buttersäure, N,N-Dimethylaminopropylacrylamid, N-Methylol (meth) acrylamid, N,N-Dimethyl-N-methacrylamidopropyl-N-(3-sulfopropyl)ammonium-betain oder 10-Acrylamidoundecansäure, (Meth)Acrylsäureester wie Hydroxyethyl(meth)acrylat, Hydroxypropyl(meth)acrylat, Ethoxy- und Methoxypolyethylenglykolmethacrylate mit 2 bis 50 Ethylenoxyeinheiten im Molekül, Polyethylenglykolmonomethacrylat, Polypropylenglykolmonomethacry- lat, Monomethacroyloxyethylphthalat, Monomethacroyloxyethylsuccinat, Butandiolmonoacrylat, Dimethylaminoethyl(meth)acrylat, Diethylaminoethyl(meth)acrylat, quaternierte Ammonium (meth) acrylate, z.B. N-Trimethyl- oder N-Triethylammoniumethyl(meth)acrylat, N-Vinylpyrrolidon, N-Vinylcaprolactam, N-Vinylimidazol, N-C₁-C₁₀-Alkyl-N'-vinylimidazolium-Verbindungen, z.B. N-Methyl-, N-Ethyl- oder N-Butyl-N'-vinylimidazolium-chlorid, -bromid oder -methosulfat, 1-Vinyl-2-methylvinylimidazol, 4- und 2-Vinylpyridin oder N-Vinylformamid. Besonders bevorzugt sind Acrylsäure, Methacrylsäure, Crotonsäure, Malein- oder Fumarsäure-C₁-bis C₁₀-alkyl-halbester, Hydroxyalkyl(meth)acrylate mit jeweils 1 bis 10 Kohlenstoffatomen in der Alkylkette, N-Tri-C₁- bis C₄-alkyl-ammonium-C₁- bis Cio-alkylacrylate oder -methacrylate, N-Vinylpyrrolidon, N-Vinylimidazol, N-C₁- bis C₁₀-Alkyl-N'-vinylimidazolium-Verbindungen, N-Vinylcaprolactam oder Mischungen hieraus.

Werden Carbonsäuren, Sulfonsäuren oder Phosphonsäuren als Monomere B eingesetzt, kann ein Teil oder die gesamte Menge dieser Monomere durch Zugabe einer Base in die entsprechenden Salze übergeführt werden. Geeignete Basen sind z.B. Alkalibasen wie Alkalimetallhydroxide und -carbonate, beispielsweise NaOH, KOH und Natrium- und Kaliumcarbonat, Ammoniak und organische Amine, Pyridine und Amidine oder Mischungen daraus. Bei der Neutralisation mit Hilfe organischer Amine verwendet man vorzugsweise Alkanolamine aus der Reihe der Mono-, Di- oder Trialkanolamine mit 2 bis 5 C-Atomen im Alkanolrest wie Mono-, Di- oder Triethanolamin, Mono-, Di- oder Tri(iso)propanolamin oder 2-Amino-2-methyl-propanol, Alkandiolamine mit 2 bis 4 C-Atomen im Alkandiolrest wie 2-Amino-2-methyl-1,3-propandiol oder 2-Amino-2-ethyl-1,3-propandiol, Alkanpolyolamine wie Tetrahydroxypropylethylendiamin, Alkylamine wie Di(2-ethylhexyl)amin, Triamylamin oder Dodecylamin und Aminoether wie Morpholin. Der Zusatz der Base kann vor, während oder nach der Polymerisation erfolgen.

Werden stickstoffhaltige, basische, ethylenisch ungesättigte Monomere B eingesetzt, so können diese vor, während oder nach der Polymerisation durch Zugabe eines Teils oder einer äquivalenten Menge einer anorganischen oder organischen Säure oder geeigneter Alkylierungsmittel, z.B. von Alkylchloriden, Alkylbromiden, Alkyliodiden oder Dialkylsulfaten, in die entsprechenden Salze oder quaternären Verbindungen übergeführt werden.

Als vernetzende Komponente C dienen olefinisch mehrfach ungesättigte Verbindungen wie beispielsweise Divinylbenzol, Divinylethylenharnstoff, Diallylweinsäurediamid, Methylenbisacrylamid, (Meth)Acrylester von mehrfunktionellen Alkoholen wie Ethylenglykol, 1,4-Butandiol, Trimethylolpropan, Pentaerythrit, Neopentylglykol, Bisphenol-A, Glycerin, Propandiol, Alkylenglykolen mit 3 bis 20 C-Atomen in der Alkylengruppe, Polyethylenglykolen oder Polypropylenglykolen, Allylester der (Meth)Acrylsäure, Trivinylcyclohexan, Triallyltriazintrion, Allylether von Trimethylolpropan, Pentaerythrit und Saccharose mit mindestens zwei Allylethereinheiten pro Molekül, Dihydrodicyclopentadienyl(meth)acrylat, N-Allylmethacrylamid, N,N-Diallylweinsäurediamin oder Diallylphthalat. Besonders bevorzugt sind Divinylbenzol, (Meth)Acrylsäureallylester, C₂- bis C₁₀-Alkylpolyol-polyacrylate oder -methacrylate, z.B. 1,4-Butandioldiacrylat oder -methacrylat, oder Mischungen hieraus.

Die Polymerisation wird unter Verwendung von in der Emulsionspolymerisation üblichen oberflächenaktiven Verbindungen als Emulgatoren bzw. Emulgatormischungen in einer Menge von 2 bis 20 Gew.-%, vorzugsweise 3 bis 18 Gew.-%, insbesondere 4 bis 15 Gew.-%, vor allem 5 bis 13 Gew.-%, bezogen auf die Gesamtmenge an eingesetzten Monomeren, durchgeführt. Im Handel erhältliche grenzflächenaktive Verbindungen werden in der Literatur in breitem Umfang beschrieben, beispielsweise bei R. Hensch, "Ullmann's Encyclopedia of Industrial Chemistry", 5. Aufl., 1987, Vol. A9, S. 297-339, bei E.W. Flick "Industrial Surfactants", Noyes Publication, Park Ridage 1988, oder bei M.R. Porter "Handbook of Surfactants", Chapman & Hall, New York 1991. Beispiele für solche Emulgatoren sind vor allem anionenaktive Verbindungen, insbesondere Alkalimetall-, Ammonium- und Aminsalze von längerkettigen Fettsäure, Alkalimetallsalze der Schwefelsäureester von Fettalkoholen und Fettalkoholethern, z.B. Natriumlaurylethersulfat mit 1 bis 15 mol Ethylenoxid, und von Alkylphenolen, Alkalimetallsalze von Sulfobernsteinsäuredialkylestern sowie Alkalimetallsalze der Sulfonsäuren von Alkylbenzolen, Alkylnaphthalinen und von Naphthalin. Es können aber auch kationenaktive Verbindungen eingesetzt werden, beispielsweise Fettamine, quartäre Ammoniumverbindungen oder quaternisierte Pyridine, Morpholine oder Imidazoline.

In vielen Fällen wird den Emulgatoren zusätzlich ein Cosolvens oder ein Cosolventiengemisch in Mengen von 0,5 bis 20 Gew.-%, insbesondere 1 bis 5 Gew.-%, zugesetzt. Bevorzugte Cosolventien sind geradkettige oder verzweigte aliphatische oder alicyclische C₁- bis C₃₀-Alkohole sowie Mischungen hieraus. Beispiele sind n-Butanol, n-Hexanol, Cyclohexanol, 2-Ethylhexanol, iso-Octanol, n-Octanol, n-Decanol, n-Dodecanol,Stearylalkohol, Oleylalkohol oder Cholesterin.

Weitere mögliche Cosolventien sind Alkandiole mit 4 bis 20 Kohlenstoffatomen im Alkylrest, Ethylenglykolalkylether mit 1 bis 4 Ethylenoxideinheiten im Molekül, N-Alkylpyrrolidone sowie N-Alkyl- und N,N-Dialkylacetamide mit jeweils 1 bis 8 Kohlenstoffatomen in der Alkylkette. Beispiele hierfür sind Ethylenglykolmonobutylether, Diethylenglykolmonoethylether, Tetraethylenglykoldimethylether, N-Methylpyrrolidon, N-Hexylpyrrolidon, Diethylacetamid oder N-Octylacetamid.

Weiterhin können bei der Polymerisation inerte, wasserunlösliche Substanzen zugegen sein, die zwar die Monomere, nicht aber die Polymerisate zu lösen vermögen. Sie wirken vielmehr als Fällungsmittel für die Polymerisate. Beispiele für diese als "Porenbildner" bezeichnete Stoffe sind lineare oder cyclische, geradkettige oder verzweigte Kohlenwasserstoffe, z.B. Cyclohexan, n-Hexan oder n-Octan, Carbonsäureester, z.B. Methylacetat, Ethylacetat, iso-Propylacetat, iso-Butylacetat oder Methylpropionat, und Ketone, z.B. Aceton, Methylethylketon oder Diethylketon, in Mengen von 1 bis 250 Gew.-%, bezogen auf die Gesamtmenge der Monomeren. Sie werden zumeist während oder nach der Reaktion durch Destillation oder ein anderes geeignetes Verfahren aus dem Reaktionsgemisch entfernt.

Die wäßrigen Copolymerisatdispersionen werden zweckmäßigerweise dadurch hergestellt, daß man die Monomeren A bis C in Wasser oder wäßrigem Medium in Gegenwart der Emulgatoren und Coemulgatoren durch Anwendung thermisch oder photochemisch Radikale bildender Initiatoren oder ionisierender Strahlung umsetzt. Die Umsetzung kann nach verschiedenen, an sich in der Emulsionspolymerisation bekannten Verfahren erfolgen. Dabei können alle Komponenten zu Beginn der Reaktion vorgelegt werden. Einzelne oder alle Komponenten können aber auch im Verlauf der Umsetzung in unter Umständen unterschiedlichen Zeiträumen partiell oder vollständig zudosiert werden. Die Zudosierung kann getrennt oder gemeinsam und gegebenenfalls aus gerührten Gefäßen heraus erfolgen.

Die Polymerisation erfolgt üblicherweise in einem gerührten Reaktionsgefäß unter einer Inertgasatmosphäre. Die Temperatur der Reaktionsmischung beträgt während der Polymerisation im allgemeinen 40 bis 160°C, vorzugsweise 50 bis 120°C. Im Falle der Umsetzung mit photochemisch wirksamen Initiatoren oder ionisierender Strahlung kann die Temperatur auch außerhalb dieses Bereichs liegen, beispielsweise zwischen 0 und 120°C. Die Temperatur kann während der Reaktion nach einem Programm unterschiedlich gesteuert werden. Der Druck im Reaktionsgefäß wird üblicherweise auf Werte von 0,8 bis 5 bar eingestellt, bevorzugt wird Atmosphärendruck.

Üblicherweise wird die Copolymerisation in Wasser durchgeführt. Es können aber auch vor, während oder nach der Umsetzung bis zu 80 Gew.-%, bezogen auf die der Wasserphase, eines niederen Alkohols wie Methanol, Ethanol oder Isopropanol oder eines niederen Ketons wie Aceton zugesetzt werden.

Die Copolymerisation erfolgt bevorzugt in Gegenwart von Initiatoren, die unter den Polymerisationsbedingungen Radikale bilden. Als Radikale bildende Initiatoren kommen alle üblichen Peroxy-und Azoverbindungen in Betracht wie Peroxide, Hydroperoxide und Peroxyester, z.B. Wasserstoffperoxid, Natrium-, Kalium- und Ammoniumperoxidisulfat, Dibenzoylperoxid, Di-tert.-butylperoxid, tert.-Butylhydroperoxid, tert.-Butylperpivalat und tert.-Butylperoxy-2-ethylhexanoat, sowie Azoverbindungen, beispielsweise 2,2' -Azobis(2-amidinopropan) dihydrochlorid, 2,2'-Azobis[2-(2-imidazolin-2-yl)propan] dihydrochlorid, 2,2' -Azobisisobutyronitril, 2,2'-Azobis(2,4-dimethyl-valeronitril), Dimethyl-2,2'-azobisisobutyrat, 2,2'-Azobis-(2-methylbutyronitril) und 2,2'-Azobis(4-methoxy-2,4-dimethylvaleronitril). Man kann selbstverständlich auch Initiatormischungen oder die bekannten Redoxinitiatoren verwenden. Diese Initiatoren werden in üblichen Mengen eingesetzt, beispielsweise 0,05 bis 5 Gew.-%, bezogen auf die zu polymerisierenden Monomere.

Die Copolymerisation kann auch durch Bestrahlung der Reaktionsmischung mit ultraviolettem Licht durchgeführt werden. Dazu setzt man der Mischung Photoinitiatoren zu, die unter diesen Bedingungen Radikale bilden. Geeignete Verbindungen dazu sind beispielsweise α-Diketone, z.B. Benzil oder Diacetyl, Acyloine, z.B. Benzoin, Acyloinether, z.B. Benzoinmethylether, Benzoinethylether oder Benzoinisopropylether, Thioxanthone, z.B. Thioxanthon, 2,4-Diethylthioxanthon, Thioxanthon-1-sulfonsäure oder Thioxanthon-4-sulfonsäure, Benzophenone, z.B. Benzophenon, 4,4'-Bis (dimethylamino)benzophenon oder 4,4'-Bis(diethylamino)benzophenon, Acetophenone, z.B. Acetophenon, p-Dimethylaminoacetophenon, α,α'-Dimethoxyacetoxyacetophenon, 2,2'-Dimethoxy-2-phenylacetophenon, p-Methoxyacetophenon oder 2-Methyl[4-(methylthio)phenyl]-2-morpholino-1-propan, Chinone, z.B. Anthrachinon oder 1,4-Naphthochinon, halogenierte Verbindungen, z.B. Phenacylchlorid, Tribrommethylphenylsulfon oder Tris(trichlormethyl)-s-triazin, sowie Peroxide, z.B. Ditert.-butylperoxid.

Diese Photopolymerisationsinitiatoren können allein oder als Mischungen von zwei oder mehreren davon eingesetzt werden und sie sind vorzugsweise in einer Menge von 0,1 bis 20 Gewichtsteilen, insbesondere 0,2 bis 10 Gewichtsteilen, bezogen auf die Summe der eingesetzten Monomere, enthalten. Diesen Photoinitiatoren können auch Aktivatoren, beispielsweise tertiäre Amine, beigegeben werden.

Das Molekulargewicht der Copolymerisate wird vorzugsweise durch Zugabe von schwefelorganischen Verbindungen als Regler zur Polymerisationsmischung kontrolliert, d.h. erniedrigt. Geeignete Regler sind beispielsweise Mercaptoverbindungen wie Dodecylmercaptan, Thioethanol, 2-Ethylhexylthioglykolat, 2-Mercapto1-ethanol oder Thioglykolsäure. Falls Regler mitverwendet werden, beträgt die Einsatzmenge normalerweise 0,1 bis 5 Gew.-%, bezogen auf die eingesetzten Monomeren.

Die wäßrigen Copolymerisatdispersionen können nach der Reaktion weiteren Verfahrensschritten unterworfen werden, um beispielsweise etwaige Gehalte an unerwünschten Verunreinigungen zu reduzieren. Solche Verfahrensschritte können beispielsweise Nachpolymerisationsverfahren, Wasserdampfbehandlungen, teilweises Abdestillieren des Lösungsmittels oder Strippverfahren sein. Sie können auch weiteren physikalischen Behandlungsschritten wie Filter-, Trocken- oder Granulierverfahren unterworfen werden.

Den wäßrigen Copolymerisatdispersionen können auch weitere übliche Hilfsmittel zugesetzt werden, beispielsweise Komplexbildner, Schaumdämpfer, Konservierungsmittel, Korrosionsinhibitoren, Radikalfänger oder Oxidationsschützmittel.

Die Lichtdurchlässigkeit (LD) einer 1 cm dicken Schicht der Copolymerisatdispersionen beträgt üblicherweise für weißes Licht bei einer Konzentration von 0,5 Gew.-% mindestens 50 %, meist werden Werte von über 70 % erreicht.

Durch die geringe Teilchengröße der Polymeren sind diese Formulierungen optisch transparent. Sie besitzen daduch anwendungstechnische Vorteile gegenüber Systemen, die mittels herkömmlicher Dispersionen formuliert werden und durch die größeren Teilchendurchmesser milchiges oder sogar undurchsichtiges Aussehen besitzen.

Die Copolymerisatdispersionen werden Filmbildner in haarkosmetischen Zubereitungen eingesetzt. Durch die geringe Teilchengröße der Polymerisate ist es in vielen Fällen möglich, transparente Formulierungen in wäßrigen oder überwiegend wäßrigen Medien bereitzustellen.

Als Haarfestigungsmittel in Haarsprays können die erfindungsgemäßen Copolymerisatdispersionen entweder in Aerosolzubereitungen zusammen mit Treibmitteln wie Dimethylether, Propan, Butan und Mischungen derselben oder als Druckluft- bzw. Pumpspray eingesetzt werden. Die feinteiligen Dispersionen erzeugen während der Trocknungsphase auf dem Haar kein grauweißes Aussehen, was bisher die Anwendung von Dispersionen in Haarsprayzubereitungen verhindert hat, weil dies vom Verbraucher nicht akzeptiert wurde. Die auf dem Haar gebildeten Filme sind klar und nicht klebrig, zeigen eine geringe Wasseraufnahme bei guter Auswaschbarkeit und bewirken eine hohe Festigung. Die Treibgasverträglichkeit ist ebenfalls gut.

Vorteil dieser Dispersionen gegenüber klassischen alkoholischen bzw. wäßrig-alkoholischen Lösungen ist die verminderte Viskosität bei hoher Wirkstoffkonzentration. Auf diese Weise gelingt insbesondere eine Reduzierung der flüchtigen organischen Verbindungen bezogen auf die eingesetzte Polymermenge. Werden übliche Polymerisate als alkoholische bzw. wäßrig-alkoholische Lösungen in die Haarsprayzubereitungen eingearbeitet, so limitiert die Viskosität die Wirkstoffkonzentration. Hochviskose Lösungen lassen sich nicht hinreichend feinteilig versprühen, so daß die Polymerkonzentration in Haarsprayzubereitungen 10 Gew.-% üblicherweise nicht übersteigt.

Gegenstand der vorliegenden Erfindung ist die Verwendung von wäßrigen Polymerisatdispersionen, die durch radikalinitiierte oder durch Anwendung ionisierender Strahlung initiierte Polymerisation in wäßrigem Medium erhältlich sind, mit einer mittleren Teilchengröße von 10 bis 30 nm, bestimmt durch Lichtstreuung in wäßrigem Medium, als Filmbildner in haarkosmetischen Zubereitungen, wobei diese Verwendung dadurch gekennzeichnet ist, daß diese kosmetische Zubereitungen einen Feststoffgehalt an diesen Polymerisaten von 11 bis 30 Gew.-%, insbesondere von 15 bis 25 Gew.-% aufweisen.

Hierbei werden wäßrige Polymerisatdispersionen eingesetzt, die durch Polymerisation von
A) 40 bis 99 Gew.-% eines oder mehrerer wasserunlöslicher, monoethylenisch ungesättigter Monomeren A,
B) 1 bis 60 Gew.-% eines oder mehrerer wasserlöslicher, monoethylenisch ungesättiger Monomeren B und
C) 0 bis 30 Gew.-%, insbesondere 0 bis 20 Gew.-%, eines oder mehrerer mehrfach ethylenisch ungesättigter Monomeren C
erhältlich sind.

Die Copolymerisatdispersionen können in Kombination mit einer oder mehreren UV-absorbierenden Komponenten eingesetzt werden. Diese Mischungen besitzen oft eine höhere Extinktionswirkung als sich aus Addition der diesen Mischungen zugrundeliegenden Komponenten ergibt. Bevorzugte Gewichtsverhältnisse von Copolymerisaten und UV-aktiven Komponenten beträgt 1:99 bis 99:1, ganz besonders bevorzugt 10:90 bis 90:10.

Geeignete UV-aktive Komponenten besitzen ein Absorptionsmaximum im Wellenlängenbereich von 200 bis 350 nm mit einer Extinktion von E > 100, gemessen in 1 gew.-%iger Lösung bei einer Schichtdicke von 1 cm. Geeignete Substanzen sind z.B. 2-Hydroxy-4-methoxybenzophenon, 2-Hydroxy-4-methoxy-benzophenon-5-sulfonsäure, 2,2' -Dihydroxy-4-methoxy-benzophenon, p-Methoxyzimtsäure, Ethyl-4-bis(2-hydroxypropyl)-aminobenzoat, Glycerin-1-(4-aminobenzoat), Homomenthylsalicylat, Menthyl-o-aminobenzoat, 2-Ethylhexyl-2-cyano-3,3-diphenyl-acrylat, 2-Ethylhexyl-p-dimethylaminobenzoat, 2-Ethylhexyl-p-methoxyzimtsäureester, 2-Ethylhexyl-salicylat, 4-Aminobenzoesäure, 2-Phenyl-benzimidazol-5-sulfonsäure, Triethanolamin-salicylat, 3-(4'-Methylbenzyliden)-bornan-2-on, 2,4-Dihydroxybenzophenon, 2,2',4,4'-Tetrahydroxybenzophenon, 2,2' -Dihydroxy-4,4'-dimethoxy-benzophenon, 2-Hydroxy-4-(2-ethylhexyloxy)-benzophenon, 1-(4'-Isopropylphenyl) -3-phenyl-propan-1,3-dion, 1-(4'-Butylphenyl)-3-(4-methoxyphenyl)-propan-1,3-dion, Ethyl-2-cyano-3,3'-diphenyl-acrylat, Octyltriazon, p-Aminobenzoesäurepolyethylenglykol-ester, Titandioxid oder Zinkoxid.

Der große Vorteil der Copolymerisatdispersionen gegenüber klassischen alkoholischen oder wäßrig-alkoholischen Lösungen ist die verminderte Viskosität bei hoher Wirkstoffkonzentration. Auf diese Weise gelingt insbesondere eine Reduzierung der flüchtigen organischen Verbindungen, bezogen auf die eingesetzte Polymermenge. Die erfindungsgemäßen Copolymerisatdispersionen weisen üblicherweise Viskositäten von 2 bis 50 mPas, insbesondere 3 bis 30 mPas, bei Feststoffgehalten von 20 bis 50 Gew.-% auf im Vergleich zu Werten von ca. 1000 bis ca. 10 000 mPas bei analogen Lösungen solcher Copolymerisate mit vergleichbaren Feststoffgehalten.

Die Copolymerisatdispersionen weisen den angestrebten hohen Feststoffgehalt und eine erniedrigte Emulgatorkonzentration auf und zeigen dabei gleichzeitig in haarkosmetischen Zubereitungen, hervorragende anwendungstechnische Ergebnisse, was auf das besondere Teilchengrößenspektrum zurückzuführen ist.

Die erfindungsgemäßen Copolymerisatdispersionen können durch den Zusatz einer Base teilweise oder vollständig neutralisiert sein. Als Base kommen in Betracht Alkali, insbesondere NaOH, Ammoniak, Amine und/oder Hydroxyamine, insbesondere 2-Amino-2-methylpropanol.

### Herstellungsbeispiele

### Beispiel 1

In einer Rührapparatur wurden 900 g VE-Wasser, 172 g einer 27 gew.-%igen wäßrigen Lösung eines mit zwei Mol Ethylenoxid hergestellten Natrium-laurylethersulfats und 8 g n-Butanol auf 70-80°C erwärmt und unter Rühren und leichtem Stickstoff-Strom mit 2 g 2,2'-Azobis(2-amidinopropan)dihydrochlorid versetzt. Zu dieser Lösung wurde ein Zulauf von 383,3 g VE-Wasser, 44 g der Nalaurylethersulfat-Lösung, 2 g n-Butanol, 45 g Methacrylsäure und 255 g t-Butylacrylat während 75 min bei einer Innentemperatur von 77°C zugetropft. Danach wurden 10 g 2-Amino-2-methyl-1-propanol in 5 min bei einer Temperatur von 77°C zugetropft. Der Ansatz wurde über eine Filternutsche abgefüllt. Die durchscheinende Dispersion besaß einen Feststoffgehalt (FG) von 20,2 Gew.-%; K-Wert (1 Gew.-% in Ethanol): 44,7; LD-Wert (weißes Licht, 0,1 Gew.-%) 98,0 % und mittlere Teilchengröße: 17,5 nm.

### Vergleichsbeispiel 1

In einer Rührapparatur wurden 350 ml VE-Wasser, 74,0 g der Natrium-laurylethersulfat-Lösung aus Beispiel 1, 2 g n-Butanol und 0,9 g Natriumperoxodisulfat unter Rühren und leichtem Stickstoffstrom auf 80°C erwärmt. Zu dieser Lösung wurde ein Zulauf von 150 ml VE-Wasser, 13,5 g der Natrium-laurylether-sulfat-Lösung, 2,0 g n-Butanol, 190 g Methylmethacrylat, 5,0 g Methacrylsäure, 10,0 g Methacrylsäureallylester und 1,3 g Ethylhexylthioglykolat während 2 1/2 h und gleichzeitig ein zweiter Zulauf von 21,5 g Methacrylsäure in 1 1/2 h zugetropft. Anschließend polymerisierte die Mischung noch 3 h nach und wurde dann über ein Leinentuch abfiltriert. Es entstand eine durchscheinende Dispersion mit FG: 30,6 Gew.-%; LD-Wert (0,01 Gew.-%) : 99 %; LD-Wert (0,5 Gew.-%): 75 % und mittlerer Teilchengröße: 36,7 nm.

### Beispiel 2

In einer Rührapparatur wurden 360 ml VE-Wasser, 60 g einer 28 gew.-%igen wäßrigen Lösung eines Natrium-laurylether-sulfats mit 2 Ethylenoxid-Einheiten im Molekül, 2,0 g n-Butanol und 1,0 g Natriumperoxodisulfat unter Rühren und leichtem Stickstoffstrom auf 80°C erwärmt. Zu dieser Lösung wurde ein Zulauf von 150 ml Wasser, 8 g der Natrium-laurylether-sulfat-Lösung, 2,0 g n-Butanol, 126 g Styrol, 18 g Acrylsäure und 6 g Divinylbenzol während 3 h zugetropft. Der Versuch wurde weitere 3 h bei dieser Temperatur gehalten. Anschließend wurde die Mischung über ein Leinentuch abfiltriert. Es entstand eine durchscheinende Dispersion mit FG: 23,2 Gew.-%; LD-Wert (0,5 Gew.-%) : 89 % und mittlerer Teilchengröße: 20,5 nm.

### Beispiel 3

In einer Rührapparatur wurden 250 ml VE-Wasser, 74 g einer 27 gew.-%igen wäßrigen Lösung eines Natrium-laurylether-sulfats mit 2 Ethylenoxid-Einheiten im Molekül, 2,0 g n-Butanol und 0,6 g Natriumperoxodisulfat unter Rühren und leichtem Stickstoffstrom auf 83°C erwärmt. Zu dieser Lösung wurde ein Zulauf von 150 ml VE-Wasser, 18,5 g der Natrium-laurylether-sulfat-Lösung, 2,0 g n-Butanol, 50 g tert.-Butylacrylat, 25 g Methacrylsäure und 50 g Methylmethacrylat während 75 min zugetropft. Der Versuch wurde weitere 2 h bei dieser Temperatur gehalten. Die Mischung wurde zuletzt über ein Leinentuch abfiltriert. Es entstand eine bläulich schimmernde, durchscheinende Flüssigkeit mit FG: 21 Gew.-%; LD-Wert (0,5 Gew.-%): 96,5 %, K-Wert (1 Gew.-%, Ethanol): 62,7 und mittlerer Teilchengröße: 16,3 nm.

### Beispiel 4

In einer Rührapparatur wurden 900 g VE-Wasser, 172 g einer 27 gew.-%igen wäßrigen Lösung eines Natrium-laurylether-sulfats mit 2 Ethylenoxid-Einheiten im Molekül, 8 g n-Butanol und 2,5 g 2,2'-Azobis(2-amidinopropan)dihydrochlorid unter Rühren und leichtem Stickstoffstrom auf 80°C erwärmt. Zu dieser Lösung wurde ein Zulauf von 383 g VE-Wasser, 44 g der Natrium-laurylether-sulfat-Lösung, 2,0 g n-Butanol, 240 g tert.-Butylacrylat und 60 g Methacrylsäure während 60 min zugetropft. Nach 25 min bei dieser Temperatur werden 10 g 2-Amino-2-methyl-1-propanol binnen 10 min zugetropft. Die Mischung wurde zuletzt über ein Leinentuch abfiltriert. Es entstand eine bläulich schimmernde, durchscheinende Flüssigkeit mit FG: 20,4 Gew.-%; LD-Wert (0,5 Gew.-%): 96,5 %, K-Wert (1 Gew.-%, Ethanol): 36,5 und mittlerer Teilchengröße: 15,0 nm.

### Beispiel 5

In einer Rührapparatur wurden 700 g VE-Wasser, 140 g einer 27 gew.-%igen wäßrigen Lösung eines Natrium-laurylether-sulfats mit 2 Ethylenoxid-Einheiten im Molekül, 4,0 g n-Butanol und eine Lösung von 0,9 g Ammoniumperoxodisulfat in 8 g VE-Wasser unter Rühren und leichtem Stickstoffstrom auf 80°C erwärmt. Zu dieser Lösung wurde ein Zulauf von 300 ml VE-Wasser, 37 g der Natriumlaurylether-sulfat-Lösung, 4,0 g n-Butanol, 5 g Methacrylsäure und 200 g Methylmethacrylat während 3 Stunden zugetropft. Dabei tropfte in diesen Zulauf während 2,75 Stunden ein weiterer Zulauf aus 45 g Methacrylsäure. Der Versuch wurde weitere 0,5 h bei dieser Temperatur gehalten. Die Mischung wurde zuletzt über ein Leinentuch abfiltriert. Es entstand eine bläulich schimmernde, durchscheinende Flüssigkeit mit FG: 20,5 Gew.-%; LD-Wert (0,5 Gew.-%): 96 % und mittlerer Teilchengröße: 17,1 nm.

### Vergleichsbeispiel 2

In einer Rührapparatur wurden 340 ml VE-Wasser, 90 g der Natrium-laurylethersulfat-Lösung aus Beispiel 1, 2 g n-Butanol und 1 g Natriumperoxodisulfat unter Rühren und leichtem Stickstoffstrom auf 80°C erwärmt. Zu dieser Lösung wurde ein Zulauf von 150 ml VE-Wasser, 15 g der Natrium-laurylether-sulfat-Lösung, 2,0 g n-Butanol, 140 g Styrol, 10 g Methacrylsäure, 6 g Divinylbenzol und 234 g Cyclohexan während 3 h zugetropft. Anschließend polymerisierte die Mischung noch 3 h nach. Das Cyclohexan wurde dann durch Destillation abgetrennt. Nach dem Erkalten wurde die Mischung über ein Leinentuch abfiltriert. Es entstand eine durchscheinende Dispersion mit FG: 26,5 Gew.-%; LD-Wert (0,5 Gew.-%): 73 % und mittlerer Teilchengröße: 34,5 nm.

### Beispiel 6

In einer Rührapparatur wurden 350 ml VE-Wasser, 33,6 g einer 28 gew.-%igen wäßrigen Lösung eines Natrium-laurylether-sulfats mit 2 Ethylenoxid-Einheiten im Molekül, 2,0 g n-Butanol und 1,0 g Natriumperoxodisulfat unter Rühren und leichtem Stickstoffstrom auf 80°C erwärmt. Zu dieser Lösung wurde ein Zulauf von 150 ml Wasser, 3,9 g der Natrium-laurylether-sulfat-Lösung, 2,0 g n-Butanol, 90 g Styrol, 36 g Ethylacrylat, 18 g Methacrylsäure und 6 g Divinylbenzol während 3 h zugetropft. Der Versuch wurde weitere 3 h bei dieser Temperatur gehalten. Anschließend wurde die Mischung über ein Leinentuch abfiltriert. Es entstand eine durchscheinende Dispersion mit FG: 21,2 Gew.-%; LD-Wert (0,5 Gew.-%): 88 % und mittlerer Teilchengröße: 23,4 nm.

### Beispiel 7

In einer Rührapparatur wurden 350 ml VE-Wasser, 54 g einer 15 gew.-%igen wäßrigen Lösung von Natrium-dodecylbenzol-sulfonat, 2,0 g n-Butanol und 1,0 g Natriumperoxodisulfat unter Rühren und leichtem Stickstoffstrom auf 80°C erwärmt. Zu dieser Lösung wurde ein Zulauf von 150 ml Wasser, 6 g der Natrium-dodecyl-benzol-sulfonat-Lösung, 2,0 g n-Butanol, 126 g Styrol, 18 g Methacrylsäure und 6 g Divinylbenzol während 3 h zugetropft. Der Versuch wurde weitere 3 h bei dieser Temperatur gehalten. Anschließend wurde die Mischung über ein Leinentuch abfiltriert. Es entstand eine durchscheinende Dispersion mit FG: 22,8 Gew.-%; LD-Wert (0,5 Gew.-%): 83 % und mittlerer Teilchengröße: 26,4 nm.

### Beispiel 8

In einer Rührapparatur wurden 350 ml VE-Wasser, 33,6 g einer 27 gew.-%igen wäßrigen Lösung eines Natrium-laurylether-sulfats mit 2 Ethylenoxid-Einheiten im Molekül, 2,0 g n-Butanol und 1 g Natriumperoxodisulfat unter Rühren und leichtem Stickstoffstrom auf 80°C erwärmt. Zu dieser Lösung wurde ein Zulauf von 150 ml VE-Wasser, 3,9 g der Natrium-laurylether-sulfat-Lösung, 2,0 g n-Butanol, 102 g tert.-Butylacrylat, 30 g Methacrylsäure, 15 g Ethylacrylat und 3 g 1,4-Butandioldiacrylat während 75 min zugetropft. Der Versuch wurde weitere 2 h bei der Temperatur gehalten. Die Mischung wurde zuletzt über ein Leinentuch abfiltriert. Es entstand eine bläulich schimmernde, durchscheinende Flüssigkeit mit FG: 22,5 Gew.-%; LD-Wert (0,5 Gew.-%): 90 % und mittlerer Teilchengröße: 23,2 nm.

### Beispiele 9 bis 13

Die Beispiele 9 bis 13 wurden in Analogie zu Beispiel 8 durchgeführt. Die Zusammensetzungen und die relevanten Eigenschaften dieser Dispersionen sowie zur besseren Übersicht nochmals die Zusammensetzungen und die relevanten Eigenschaften der vorangehenden Beispiele sind in nachfolgender Tabelle zusammengefaßt.

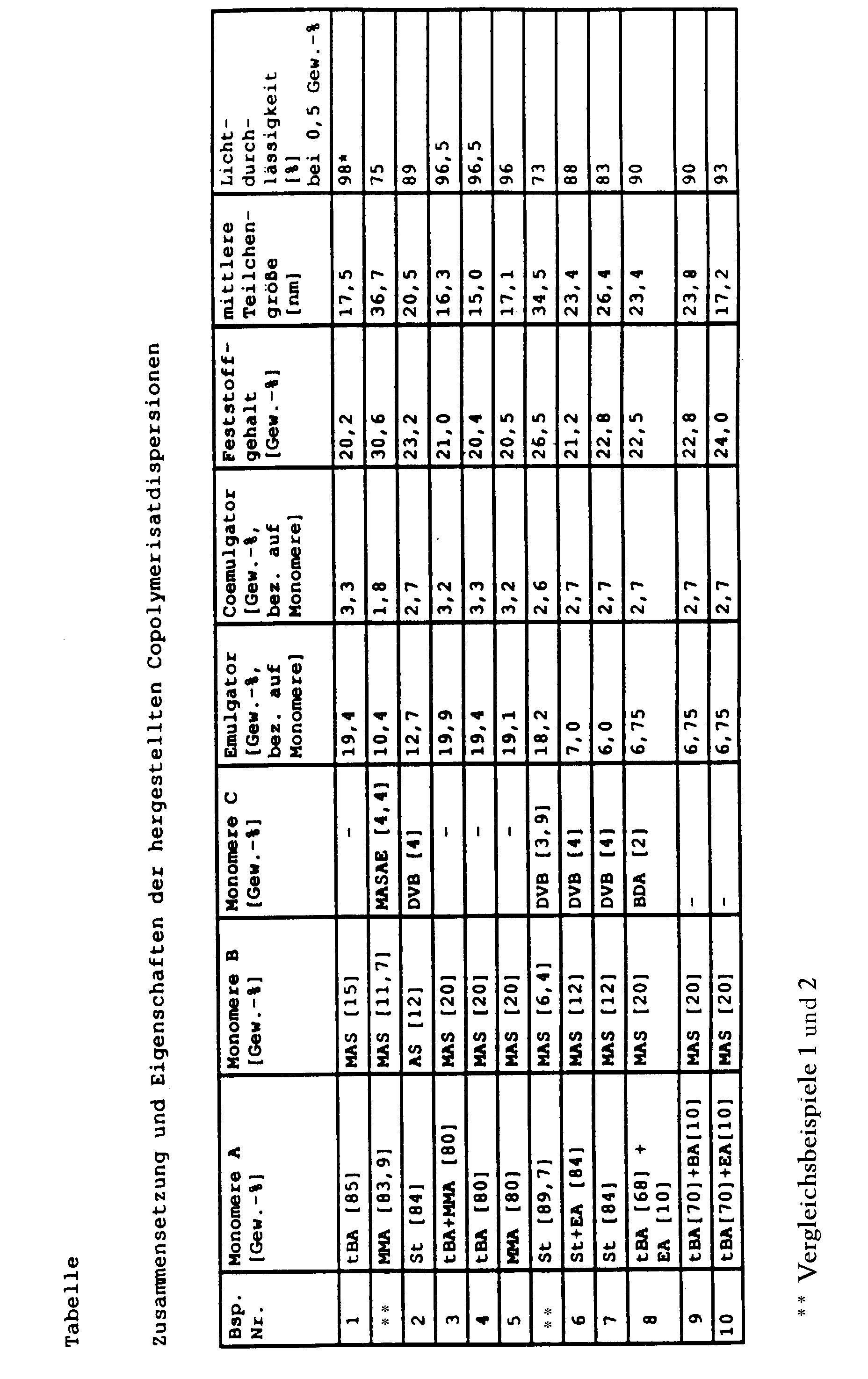

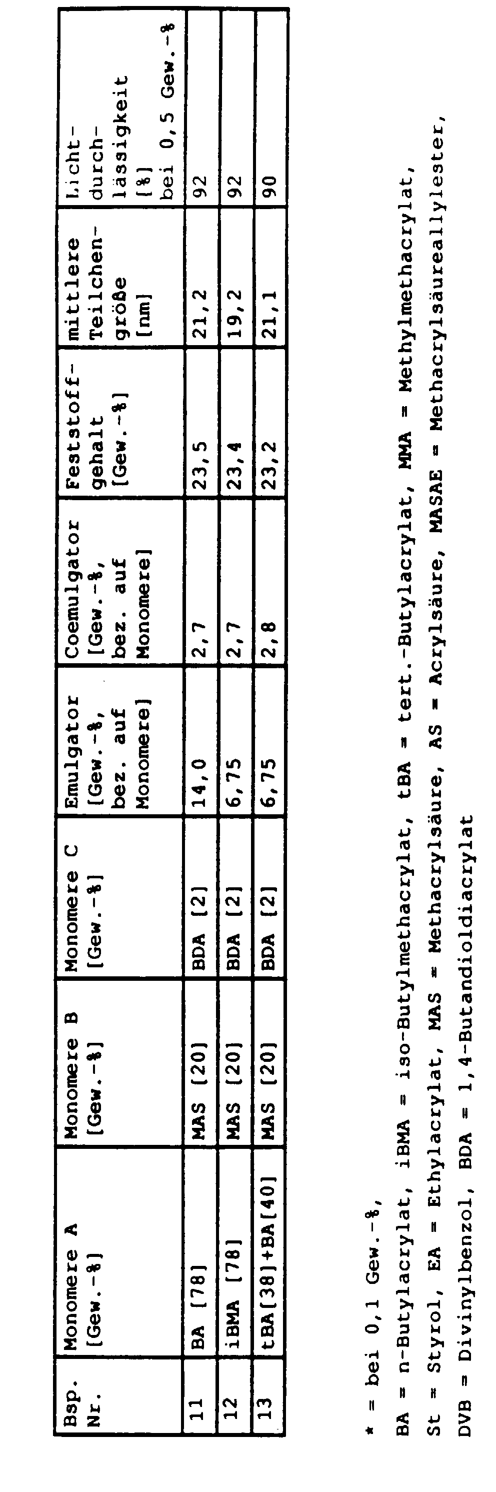

### Anwendungstechnische Beispiele

### Beispiel 14

Mit der Copolymerisatdispersion aus Beispiel 4 wurde eine stark festigende Aerosolhaarsprayformulierung hergestellt (70 g der Dispersion mit 2-Amino-2-methylpropanol auf pH 7,5 eingestellt + 30 g Dimethoxyethan). Diese Mischung ließ sich gut versprühen (niedrige Viskosität), bildete einen klaren Film und zeigte keinen Weißeffekt, d.h. war auf dunklem Haar nicht zu sehen.

### Beispiel 15

Mit der Copolymerisatdispersion aus Beispiel 4 wurde eine stark festigende Pumphaarsprayformulierung hergestellt (100 g der Dispersion mit 2-Amino-2-methylpropanol auf pH 7,5 eingestellt). Die Probe konnte direkt als ca. 20 gew.-%ige Formulierung versprüht werden (feiner Sprühstrahl) und zeigte keinen Weißeffekt,

### Beispiel 16

Mit der Copolymerisatdispersion aus Beispiel 3 wurde eine Aerosolhaarsprayformulierung hergestellt (25 g der Dispersion mit 2-Amino-2-methylpropanol auf pH 6,5 eingestellt + 25 g Ethanol + 20 g Wasser + 30 g Dimethylether). Diese Mischung zeigte eine ausgezeichnete Festigungswirkung, ließ sich gut versprühen und zeigte keinen Weißeffekt.

### Beispiele 17 bis 22

Analog zu Beispiel 15 wurden mit den Dispersionen aus den Beispielen 8 bis 13 stark festigende Pumphaarsprayformulierungen hergestellt. Alle Proben konnten direkt als ca. 22 bis 24 gew.-%ige Formulierungen versprüht werden (feiner Sprühstrahl) und zeigten keinen Weißeffekt.

## Patentansprüche

1. Verwendung von wäßrigen Copolymerisatdispersionen, erhältlich durch radikalinitiierte oder durch Anwendung ionisierender Strahlung initiierte Copolymerisation von
A) 40 bis 99 Gew.-% eines oder mehrerer wasserunlöslicher, monoethylenisch ungesättigter Monomeren,
B) 1 bis 60 Gew.-% eines oder mehrerer wasserlöslicher, monoethylenisch ungesättigter Monomeren und
C) 0 bis 30 Gew.-% eines oder mehrerer mehrfach ethylenisch ungesättigter Monomeren
in wäßrigem Medium in Gegenwart von 2 bis 20 Gew.-%, bezogen auf die Gesamtmenge der Monomeren, oberflächenaktiver Verbindungen als Emulgatoren, mit einer mittleren Teilchengröße von 10 bis 30 nm, bestimmt durch Lichtstreuung in wäßrigem Medium, und einem Feststoffgehalt von 20 - 50 Gew.-%, als Filmbildner in haarkosmetischen Zubereitungen.

2. Verwendung nach Anspruch 1, wobei die wäßrigen Copolymerisatdispersionen erhältlich sind in Gegenwart von zusätzlich 0,5 bis 20 Gew.-%, bezogen auf die Gesamtmenge der Monomeren, aliphatischer oder alicyclischer Alkohole mit 1 bis 30 C-Atomen als Cosolventien.

3. Verwendung nach Anspruch 1, wobei die wäßrigen Copolymerisatdispersionen erhältlich sind in Gegenwart von zusätzlich 0,1 bis 5 Gew.-%, bezogen auf die Gesamtmenge der Monomeren, schwefelorganischer Verbindungen als Regler.

4. Verwendung nach Anspruch 1, wobei die wäßrigen Copolymerisatdispersionen erhältlich sind durch Copolymerisation von
A) 60 bis 92 Gew.-% der Monomeren A und
B) 8 bis 40 Gew.-% der Monomeren B
oder durch Copolymerisation von
A) 60 bis 93,5 Gew.-% der Monomeren A,
B) 6 bis 32 Gew.-% der Monomeren B und
C) 0,5 bis 15 Gew.-% der Monomeren C.

5. Verwendung nach Anspruch 1, wobei die wäßrigen Copolymerisatdispersionen erhältlich sind durch Copolymerisation von Styrol, Vinylestern von Alkylcarbonsäuren mit 2 bis 30 C-Atomen im Molekül, Alkylacrylaten oder -methacrylaten mit jeweils 1 bis 10 C-Atomen in der Alkylkette oder Mischungen hieraus als Monomeren A.

6. Verwendung nach Anspruch 1, wobei die wäßrigen Copolymerisatdispersionen erhältlich sind durch Copolymerisation von Acrylsäure, Methacrylsäure, Crotonsäure, Malein- oder Fumarsäure-01- bis C₁₀-alkyl-halbestern, Hydroxyalkylacrylaten oder -methacrylaten mit jeweils 1 bis 10 C-Atomen in der Alkylkette, N-Tri-C₁- bis C₄-alkylammonium-C₁- bis C₁₀-alkylacrylaten oder -methacrylaten, N-Vinylpyrrolidon, N-Vinylimi-dazol, N-C₁- bis C₁₀-Alkyl-N'-vinylimidazolium-Verbindungen, N-Vinylcaprolactam oder Mischungen hieraus als Monomeren B.

7. Verwendung nach Anspruch 1, wobei die wäßrigen Copolymerisatdispersionen erhältlich sind durch Copolymerisation von Divinylbenzol, Acrylsäure- oder Methacrylsäureallylester, C₂-bis C₁₀-Alkylpolyol-polyacrylaten oder -methacrylaten oder Mischungen hieraus als Monomeren C.

8. Verwendung nach Anspruch 1, wobei die wäßrigen Copolymerisatdispersionen erhältlich sind in Gegenwart von zusätzlich 1 bis 250 Gew.-%, bezogen auf die Gesamtmenge der Monomeren, eines Kohlenwasserstoffes, eines Carbonsäureesters, eines Ketons oder einer Mischung hieraus, wobei die Monomere A bis C in diesen Lösungsmitteln löslich sind, nicht aber die hieraus gebildeten Copolymerisate.

9. Verwendung nach Anspruch 1, wobei die wäßrigen Copolymerisatdispersionen durch Zusatz einer Base, insbesondere 2-Amino-2-methylpropanol, teilweise oder vollständig neutralisiert sind.

## Claims

1. Use of an aqueous copolymer dispersion obtainable by copolymerization, initiated by free radicals or by the use of ionizing radiation, of
A) from 40 to 99% by weight of one or more water-insoluble, monoethylenically unsaturated monomers,
B) from 1 to 60% by weight of one or more water-soluble, monoethylenically unsaturated monomers and
C) from 0 to 30% by weight of one or more polyethylenically unsaturated monomers
in an aqueous medium in the presence of from 2 to 20% by weight, based on the total amount of monomers, of surfactant compounds as emulsifiers, and having a mean particle size of from 10 to 30 nm, determined by light scattering in an aqueous medium, and having a solids content of 20-50% by weight, as a film former in formulations for hair cosmetics.

2. Use as claimed in claim 1, the aqueous copolymer dispersion being obtainable in the presence of, in addition, from 0.5 to 20% by weight, based on the total amount of the monomers, of aliphatic or alicyclic alcohols of 1 to 30 carbon atoms as cosolvents.

3. Use as claimed in claim 1, the aqueous colpolymer dispersion being obtainable in the presence of, in addition, from 0.1 to 5% by weight, based on the total amount of the monomers, of organosulfur compounds as regulators.

4. Use as claimed in claim 1, the aqueous copolymer dispersion being obtainable by copolymerizing
A) from 60 to 92% by weight of the monomers A and
B) from 8 to 40% by weight of the monomers B
or by copolymerizing
A) from 60 to 93.5% by weight of the monomers A,
B) from 6 to 32% by weight of the monomers B and
C) from 0.5 to 15% by weight of the monomers C.

5. Use as claimed in claim 1, the aqueous copolymer dispersion being obtainable by copolymerizing styrene, vinyl esters of alkanecarboxylic acids having 2 to 30 carbon atoms in the molecule, alkyl acrylates or methacrylates having in each case 1 to 10 carbon atoms in the alkyl chain or mixtures thereof as monomers A.

6. Use as claimed in claim 1, the aqueous copolymer dispersion being obtainable by copolymerizing acrylic acid, methacrylic acid, crotonic acid, C₁-C₁₀-alkyl half-esters of maleic or fumaric acid, hydroxyalkylacrylates or methacrylates having in each case 1 to 10 carbon atoms in the alkyl chain, N-tri-C₁-C₄-alkylammonium C₁-C₁₀-alkylacrylates or -methacrylates, N-vinylpyrrolidone, N-vinylimidazole, N-C₁-C₁₀-alkyl-N'-vinylimidazolium compounds, N-vinylcaprolactam or mixtures thereof as monomers B.

7. Use as claimed in claim 1, the aqueous copolymer dispersion being obtainable by copolymerizing divinylbenzene, allyl acrylates or methacrylates, C₂-C₁₀-alkylpolyol polyacrylates or polymethacrylates or mixtures thereof as monomers C.

8. Use as claimed in claim 1, the aqueous copolymer dispersion being obtainable in the presence of, in addition, from 1 to 250% by weight, based on the total amount of the monomers, of a hydrocarbon, of a carboxylic ester, of a ketone or of a mixture thereof, the monomers A to C being soluble in these solvents but the resulting copolymers being insoluble therein.

9. Use as claimed in claim 1, the aqueous copolymer dispersion being partially or completely neutralized by adding a base, in particular 2-amino-2-methylpropanol.

## Revendications

1. Utilisation de dispersions aqueuses de copolymères obtenues par copolymérisation radicalaire ou induite par des radiations ionisantes, de
A) 40 à 99 % en poids d'un ou plusieurs monomères à insaturation monoéthylénique insolubles dans l'eau,
B) 1 à 60 % en poids d'un ou plusieurs monomères à insaturation monoéthylénique solubles dans l'eau et
C) 0 à 30 % en poids d'un ou plusieurs monomères à insaturation multiéthylénique en milieu aqueux,
en présence de 2 à 20 % en poids, par rapport à la quantité totale des monomères, d'agents tensio-actifs servant d'agents émulsionnants, à une dimension de particule moyenne de 10 à 30 nm, déterminée par diffraction de la lumière en milieu aqueux, et une teneur en matières solides de 20 à 50 % en poids,
en tant qu'agents filmogènes dans des compositions cosmétiques pour la chevelure.

2. Utilisation selon la revendication 1, dans laquelle les dispersions aqueuses de copolymères sont obtenues en présence, en outre, de 0,5 à 20 % en poids, par rapport à la quantité totale des monomères, d'alcools aliphatiques ou alicycliques en C1-C30 servant de tiers-solvants.

3. Utilisation selon la revendication 1, dans laquelle les dispersions aqueuses de copolymères sont obtenues en présence, en outre, de 0,1 à 5 % en poids, par rapport à la quantité totale des monomères, de dérivés organiques du soufre servant de régulateurs.

4. Utilisation selon la revendication 1, dans laquelle les dispersions aqueuses de copolymères sont obtenues par copolymérisation de
A) 60 à 92 % en poids des monomères A et
B) 8 à 40 % des monomères B,
ou par copolymérisation de
A) 60 à 93,5 % en poids des monomères A,
B) 6 à 32 % en poids des monomères B et
C) 0,5 à 15 % en poids des monomères C.

5. Utilisation selon la revendication 1, dans laquelle les dispersions aqueuses de copolymères sont obtenues par copolymérisation du styrène, d'esters vinyliques d'acides alcanoïques contenant 2 à 30 atomes de carbone dans la molécule, d'acrylates ou méthacrylates d'alkyle contenant chacun 1 à 10 atomes de carbone dans la chaîne alkyle ou de mélanges de ces monomères, en tant que monomères A.

6. Utilisation selon la revendication 1, dans laquelle les dispersions aqueuses de copolymères sont obtenues par copolymérisation de l'acide acrylique, de l'acide méthacrylique, de l'acide crotonique, d'hémiesters alkyliques en C1-C10 de l'acide maléique ou de l'acide fumarique, d'acrylates ou méthacrylates d'hydroxyalkyle contenant chacun 1 à 10 atomes de carbone dans la chaîne alkyle, de (alkyle en C1-C10)-acrylates ou -méthacrylates de N-tri-(alkyle en C1-C4)-ammonium, de la N-vinylpyrrolidone, du N-vinylimidazole, de dérivés de N-(alkyle en C1-C10)-N'-vinylimidazolium, du vinylcaprolactame ou de mélanges de ces monomères, en tant que monomères B.

7. Utilisation selon la revendication 1, dans laquelle les dispersions aqueuses de copolymères sont obtenues par copolymérisation du divinylbenzène, de l'acrylate ou méthacrylate d'allyle, de polyacrylates ou polyméthacrylates d'(alkyle en C2-C10)-polyols ou leurs mélanges, en tant que monomères C.

8. Utilisation selon la revendication 1, dans laquelle les dispersions aqueuses de copolymères sont obtenues en présence, en outre, de 1 à 250 % en poids, par rapport à la quantité totale des monomères, d'un hydrocarbure, d'un ester d'acide carboxylique, d'une cétone ou d'un mélange de tels composés, dans lesquels les monomères A à C sont solubles mais non les copolymères formés à partir de ces monomères.

9. Utilisation selon la revendication 1, dans laquelle les dispersions aqueuses de copolymères sont neutralisées en totalité ou en partie par addition d'une base, plus spécialement le 2-amino-2-méthylpropanol.
